# EUROPEAN PATENT SPECIFICATION

(11) **EP 3 068 366 B1**
(45) Date of publication and mention of the grant of the patent: **15.07.2020**
(21) Application number: 14798846.3
(22) Date of filing: 13.11.2014
(51) Int. Cl.: A61K 8/27, A61K 8/365, A61K 8/368, A61Q 15/00, A61K 8/19, C07C 63/08

(54) **USE AS A DEODORANT AGENT OF A SALIFIED SALICYLIC ACID DERIVATIVE, ALONE OR IN A MIXTURE**
VERWENDUNG EINES SALICYLSÄUREDERIVATS IN SALZFORM, ALLEIN ODER IN EINER MISCHUNG, ALS DESODORIERUNGSMITTEL
UTILISATION COMME AGENT DÉODORANT D'UN DÉRIVÉ SALIFIÉ D'ACIDE SALICYLIQUE, SEUL OU EN MÉLANGE

(30) Priority: 13.11.2013 FR 1361080
(43) Date of publication of application: 21.09.2016
(73) Proprietor: L'OREAL, 75008 Paris (FR)
(72) Inventor: BROSSAT, Maud, F-93600 Aulnay sous Bois (FR); TOURNIER-COUTURIER, Lucie, F-93600 Aulnay sous Bois (FR)
(74) Representative: Lavoix
(86) International application number: PCT/EP2014/074522
(87) International publication number: WO 2015/071374

(56) References cited:
- EP-A1- 1 486 199
- WO-A1-2007/031117
- CN-B- 102 349 919
- JP-A- H1 036 883
- JP-A- H09 241 675
- CESAR SCORZZA ET AL: "Synthesis and Physicochemical Characterization of Anionic Surfactants Derived from Cashew Nut Shell Oil", JOURNAL OF SURFACTANTS AND DETERGENTS, vol. 13, no. 1, 27 June 2009 (2009-06-27), pages 27-31, XP055126772, ISSN: 1097-3958, DOI: 10.1007/s11743-009-1143-5
- EICHBAUM ET AL: "Biological properties of anacardic acid (o-pentadecadienylsalicylic acid) and related compounds. I. General discussion.-Bactericidal action", MEMORIAS DO INSTITUTO BUTANTAN,, vol. 19, 1 January 1946 (1946-01-01), pages 71-96, XP009178873, ISSN: 0073-9901
- FARIAS DAVI F ET AL: "INSECTICIDAL ACTION OF SODIUM ANACARDATE FROM BRAZILIAN CASHEW NUT SHELL LIQUID AGAINST AEDES AEGYPTI", JOURNAL OF THE AMERICAN MOSQUITO CONTROL ASSOCIATION : MOSQUITO NEWS, AMERICAN MOSQUITO CONTROL ASSOCIATION MOUNT LAUREL, NJ : ASSOC, US, vol. 25, no. 3, 1 September 2009 (2009-09-01), pages 386-389, XP009178855, ISSN: 8756-971X cited in the application
- K. S. NAGABHUSHANA ET AL: "Selective Ionophoric Properties of Anacardic Acid", JOURNAL OF NATURAL PRODUCTS, vol. 58, no. 5, 1 May 1995 (1995-05-01), pages 807-810, XP055126968, ISSN: 0163-3864, DOI: 10.1021/np50119a029

## Description

The present invention relates to the use of a mixture of salified salicylic acid derivatives having formula (II) defined in more detail hereinafter, for treating body odor, in particular underarm odor, particularly in a composition comprising a physiologically acceptable medium.

The present invention also describes novel salified salicylic acid derivatives having formula (IV) and (V) defined in more detail hereinafter and the compositions containing same in a physiologically acceptable medium.

The invention also relates to a method for treating body odor.

In the field of cosmetics, the use in topical application of deodorant products containing antiperspirant or deodorant type active substances for reducing or eliminating generally unpleasant underarm odor is well known.

Eccrine or apocrine sweat has a low odor when secreted. It is the degradation thereof by bacteria via enzyme reactions that produces malodorous compounds. The function of deodorant agents is that of reducing or preventing the formation of unpleasant odor.

Deodorant substances generally destroy the resident bacterial flora. Of these substances, the most commonly used are Triclosan (2,4,4'-trichloro-2'-hydroxydiphenylether) and farnesol which involve the drawback of modifying the ecology of skin flora significantly. There are substances that reduce bacterial growth. Of these substances, mention may be made of transition metal chelating agents such as EDTA or DPTA. These materials deprive the environment of the metals required for bacterial growth.

However, there remains a need to find novel ingredients suitable for being integrated in a cosmetic formulation for treating unpleasant body odor associated with human perspiration, particularly underarm odor.

There remains a need to find novel active ingredients having a deodorant activity while being easy to formulate in compositions for reducing perspiration and/or odor, particularly in humans, and more particularly for combating body odor, and more specifically underarm odor.

Some salicylic acid derivatives such as those described in the patent FR 2 581 542, the applications WO97/15278 and WO 04/073745, in particular n-octanoyl-5-salicylic acid (or capryloyl salicylic acid) manufactured under the trade name MEXORYL SAB® by CHIMEX, were proposed in the applications WO2007/031117 and WO2007/111298 as deodorant agents. However, the deodorant activity of these compounds still remains very insufficient.

In the article in Pharmaceutical Biology 2002, 40, 231-234), the mixture of non-salified anacardic acid is known for the antimicrobial activity thereof particularly in respect of C. Xerosis. The deodorant activity of such a mixture is however low.

Some 6-alkyl- or 6-akylen-salicylic acid salts and particularly metal salts of anacardic acid are known in the document J. Am. Mosquito Control Association 2009, 25, 386-389, particularly the following mixture of anacardic acid salts 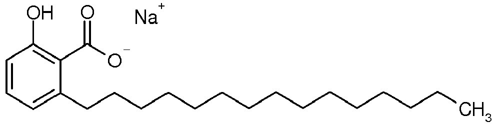 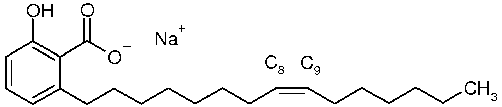 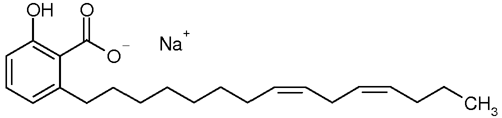 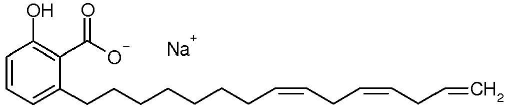 for the insecticidal properties thereof.

The document Nature 1958, 182, 1299-1300 (CMC measurement and surface activity characteristics, no mention of application on skin) and the document Phytotherapy Res. 1987, 1, 127-134 (PGE2 synthesis inhibitor), disclose the following sodium 6-pentadecylsalicylic acid salt:

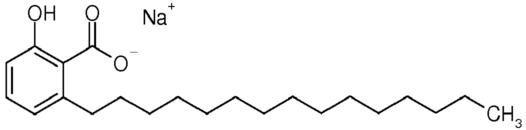

No indication is given in respect of the possibility of using these compounds as a deodorant agent particularly in a composition comprising a physiologically acceptable medium.

Some 6-alkyl- or 6-akylen-salicylic salts and particularly metal salts or amino acid salts of anacardic acid have already been used in cosmetic or dermatological compositions such as detergent products such as shampoos, photoprotection products, skin care products. These compounds are particularly used for the antibacterial properties thereof, the photoprotective effects thereof, the antioxidant effects thereof, the antiinflammatory properties thereof, for treating acne. This is the case for example in the patent applications JP10001692, JP2010059070, JP10036887 (skin cleansing), JP10036884, JP10036883, JP10036244, JP10029918, JP09241675, JP07285826, JP06329536, JP06329526, JP06329516, JP06092837, JP03240721, JP03240718, JP04036238. No indication is given in respect of the possibility of using these compounds as a deodorant agent particularly in a composition comprising a physiologically acceptable medium.

The inventors discovered surprisingly that salified salicylic acid derivatives having formula (II) detailed hereinafter, alone or in a mixture, had a good deodorant efficacy and could be readily formulated in a product intended to reduce body odor, alone or optionally in association with conventional antiperspirants or deodorant agents, without the drawbacks mentioned above.

As such, the invention is as described in the claims.

It relates to the use of compounds having formula (II), described in more detail hereinafter, in a mixture, as a deodorant agent particularly in a composition comprising a physiologically acceptable medium.

The present invention also describes novel compounds having formula (IV) or (V) described in more detail hereinafter.

The present invention also describes a composition comprising in a physiologically acceptable medium at least one compound or mixture of compounds having formula (IV) or (V) described in detail hereinafter.

The present invention also relates to a composition comprising in a physiologically acceptable medium a mixture of compounds having formula (II) in association with at least one antiperspirant agent and/or at least one additional deodorant agent.

The present invention also relates to a cosmetic method for treating body odor, particularly underarm odor, consisting of applying on the surface of a human keratin material a composition as defined above.

Further subject matter of the invention is described hereinafter. The invention is described in more detail hereinafter.

According to the present invention, the term "physiologically acceptable medium" denotes a medium suitable for administering a composition by the topical route. A physiologically acceptable medium is preferentially a cosmetically or dermatologically acceptable medium, i.e. free from odor, or unpleasant appearance, and which is perfectly compatible with the topical administration route. In the present case where the composition is intended to be administered by the topical route, i.e. by applying on the surface of the keratin material in question, such a medium is particularly considered to be physiologically acceptable when it does not give rise to stinging, tautness or redness unacceptable for the user.

The term "deodorant agent" denotes in the context of the present invention any agent, alone, with the effect of masking, absorbing, enhancing and/or reducing the unpleasant odor resulting from the decomposition of human sweat. The meaning of deodorant active substance and agent is equivalent according to the present invention.

The term "antiperspirant agent" denotes any substance, alone, with the effect of reducing sweat flow, reducing the damp sensation on the skin associated with human sweat, masking human sweat. The meaning of antiperspirant active substance and agent is equivalent according to the present invention.

The term "human keratin material" denotes in the present invention skin (body, face, eye contour), skin appendages particularly hair, eyelashes, eyebrows, and nails, and more particularly skin.

### SALIFIED SALICYLIC ACID DERIVATIVES

### a) Definitions

The salified salicylic acid derivatives according to the invention, used in a mixture, are chosen from those complying with the following formula (II), along with the tautomeric forms thereof, the optical isomers thereof, the geometric isomers thereof and the solvates thereof such as hydrates: 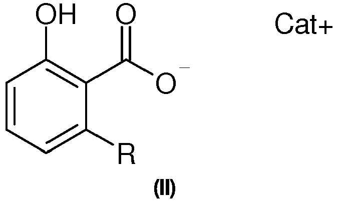 where
R denotes a saturated or unsaturated linear or branched hydrocarbon radical comprising from 12 to 20 carbon atoms, where said radical R may comprise one or a plurality of ethylene unsaturations (of the double bond type) and
Cat+ represents a cation or a mixture of cations, organic or inorganic, optionally being + or 2+, independently of the cationic charge and suitable for obtaining electroneutrality of the compound or mixture of compounds having formula (II).

According to the invention, a mixture of at least 4 compounds having formula (II), denoted compounds **(IIA), (IIB) (IIC), (IID),** is used, wherein:
- the radicals R of each of the compounds **(IIA), (IIB), (IIC), (IID)** denote a linear hydrocarbon radical comprising from 12 to 18 carbon atoms, and preferably having the same number of carbon atoms for each of the compounds **(IIA), (IIB), (IIC), (IID):**
- the compound **(IIA),** having a saturated radical R; and
- the compound **(IIB),** having a mono-unsaturated radical R; and
- the compound **(IIC),** having a di-unsaturated radical R; and
- the compound **(IID),** having a tri-unsaturated radical R; and
- Cat+ having the same meaning for each of the compounds **(IIA), (IIB) (IIC), (IID).**

Particularly preferably, R denotes a saturated or unsaturated linear C₁₄-C₁₆ hydrocarbon radical.

Advantageously, the cation Cat+ is chosen such that the compound having formula (II) is physiologically acceptable.

According to the present invention, the term "physiologically acceptable compound having formula (II)" denotes any compound having formula (II) suitable for administering a composition containing same by the topical route.

Of the inorganic cations Cat+, mention may be made of alkaline metals such as sodium (Na⁺), potassium (K⁺); alkaline-earth metals such as calcium (Ca²⁺), strontium (Sr²⁺) and magnesium (Mg²⁺); transition metals such as copper (Cu²⁺), iron (Fe²⁺) and manganese (Mn²⁺).

Of the organic cations Cat+, mention may be made of the cationic form of amines or quaternary ammoniums, and more particularly the cationic form of triethanolamine, mono-ethanolamine, di-ethanolamine, hexadecylamine, N,N,N',N'-tetrakis-(hydroxypropyl-2) ethylene di-amine and tris-hydroxymethyl aminomethane.

According to one particular form of the invention, the cation Cat+ is the cationic form of an L or D form amino acid, such as for example the cationic form of lysine, arginine, alanine, tryptophan or a quaternary ammonium N⁺R1R₂R₃-L-CO₂H, where R₁, R₂, R3, identical or different, denote a linear saturated alkyl radical comprising from 1 to 12 carbon atoms and L denotes a divalent saturated linear hydrocarbon radical comprising from 2 to 6 carbon atoms.

Advantageously in one embodiment, the inorganic cation Cat+ denotes Mg²⁺, Zn²⁺ or Cu²⁺.

Advantageously in one embodiment, the cation Cat+ denotes Na+.

Advantageously in one embodiment, the organic cation Cat+ denotes the cationic form of triethanolamine, mono-ethanolamine or di-ethanolamine,

Advantageously in one embodiment, the organic cation Cat+ denotes the cationic form of an amino acid chosen from lysine, arginine, alanine or tryptophan.

Advantageously, according to a further embodiment, the cation Cat+ denotes the cationic form of lysine.

More preferentially, a mixture of compounds **(IIA), (IIB) (IIC), (IID)** as defined above is used, wherein:
- the proportion of compound **(IIA),** varies from 0.001 to 5%,
- the proportion of compound **(IIB)** varies from 20 to 45%,
- the proportion of compound **(IIC)** varies from 5 to 35%,
- the proportion of compound **(IID)** varies from 25 to 50%, the percentages being by mass in relation to the total mass of the mixture of compounds **(IIA), (IIB) (IIC)** and **(IID).**

Preferably, a mixture of compounds having formula (II) is used, wherein:
- the proportion of compound **(IIA),** varies from 0.5 to 1%,
- the proportion of compound **(IIB)** varies from 30 to 35%,
- the proportion of compound **(IIC)** varies from 15 to 25%, the proportion of compound **(IID)** varies from 35 to 40%, the percentages being by mass in relation to the total mass of the mixture of compounds **(IIA), (IIB) (IIC)** and **(IID)**

According to one particularly preferred embodiment, a mixture of compounds **(IIA), (IIB) (IIC), (IID)** is used, wherein R denotes a hydrocarbon radical having 15 carbon atoms.

According to one particularly preferred form of the invention, the following compounds (A), (B), (C) and (D) are usedin a mixture: 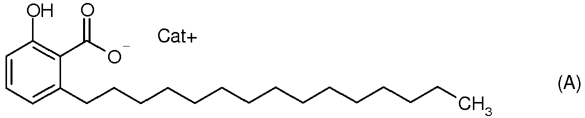 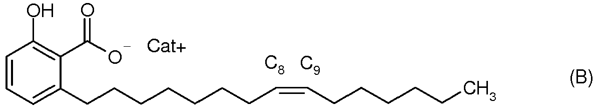 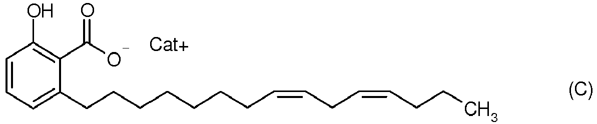 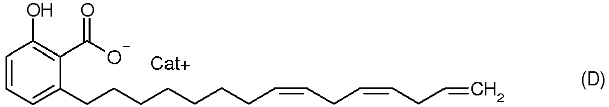 Cat+ having the meaning given above.

Particularly preferably, a specific mixture of compounds (A), (B), (C) and (D) is used, wherein:
- the proportion of compound (A) varies from 0.001 to 5%,
- the proportion of compound (B) varies from 20 to 45%,
- the proportion of compound (C) varies from 5 to 35%,
- the proportion of compound (D) varies from 25 to 50%, the percentages being by mass in relation to the total mass of the mixture of compounds (A), (B), (C) and (D).

Preferably,
- the proportion of compound (A) varies from 0.5 to 1%,
- the proportion of compound (B) varies from 30 to 35%,
- the proportion of compound (C) varies from 15 to 25%,
- the proportion of compound (D) varies from 35 to 40%.

According to one particularly preferred embodiment of the invention, of the compounds **(IIA), (IIB) (IIC), (IID),,** the following compounds **1, 2, 3, 4, 5, 6, 7, 8,** shall be usedin a mixture:

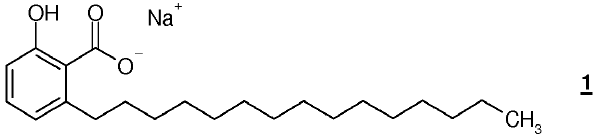

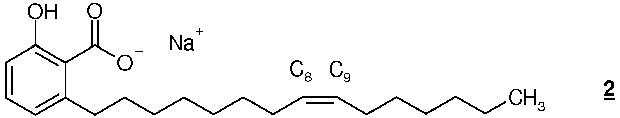

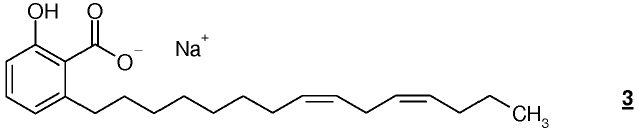

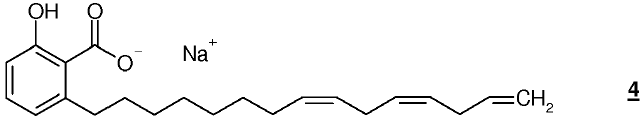

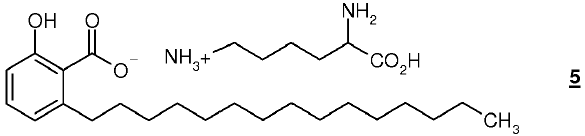

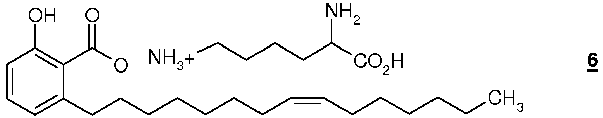

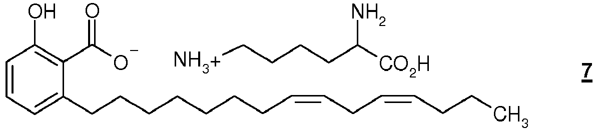

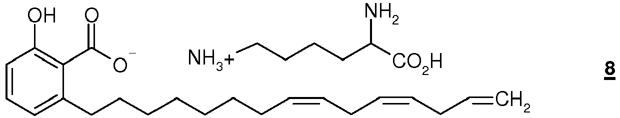

Advantageously, of the compounds **(IIA),** (**IIB**) (**IIC**), **(IID),,** a mixture of compounds 1, 2, 3 and 4 shall be used, particularly in the proportions given above for compounds (A), (B), (C) and (D) respectively, and even more preferentially a mixture comprising 5% by mass of compound 1, 35% of compound 2, 23% by mass of compound 3 and 37% by mass of compound 4.

According to a further embodiment, of the compounds (**IIA**), (**IIB**) (**IIC**), **(IID),,** a mixture of compounds 5, 6, 7 and 8 shall be used, particularly in the proportions described above for compounds (A), (B), (C) and (D) respectively, and even more preferentially a mixture comprising 5% by mass of compound 5, 35% of compound 6, 23% by mass of compound 7 and 37% by mass of compound 8.

The compound(s) having formula (II) are preferably present in quantities ranging from 0.01 to 10% by mass and more preferentially from 0.02 to 5% by mass in relation to the total mass of the composition.

### b) Novel compounds

Some compounds having formula (II) are described.

Compounds having the following formulas (IV) and (V) are described along with the tautomeric forms thereof, the optical isomers thereof, the geometric isomers thereof and the solvates thereof: 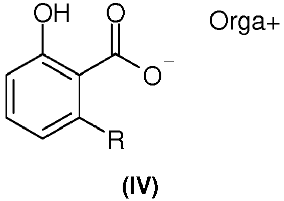 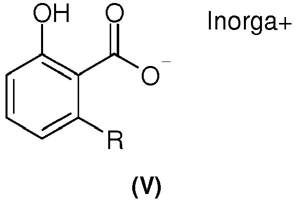 where R denotes a linear or branched hydrocarbon radical having from 12 to 20 carbon atoms (C₁₂-C₂₀), that is saturated or unsaturated (one or a plurality ethylene unsaturations), and
- Orga+ represents an organic cation chosen from the cationic form of mono-ethanolamine, di-ethanolamine, hexadecylamine, N,N,N',N'-tetrakis-(hydroxypropyl-2)ethylene di-amine or tris-hydroxymethyl aminomethane, alanine or tryptophan, a quaternary ammonium N⁺R₁R₂R₃-L-CO₂H, where R1, R2, R3, identical or different, denote a linear saturated alkyl radical comprising from 1 to 12 carbon atoms and L denotes a divalent saturated linear hydrocarbon radical comprising from 2 to 6 carbon atoms.
- Inorga+ represents an inorganic cation chosen from zinc (Zn²⁺), copper (Cu²⁺), iron (Fe²⁺), strontium (Sr²⁺), magnesium (Mg²⁺), or manganese (Mn²⁺).

As such, the compounds having the following formulae (IV) and (V) are novel along with the tautomeric forms thereof, the optical isomers thereof, the geometric isomers thereof and the solvates thereof: 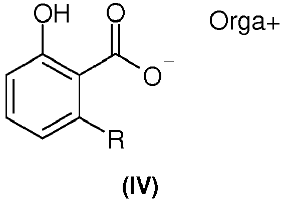 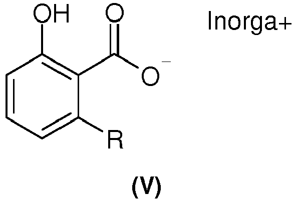 where R denotes a linear or branched hydrocarbon radical having from 12 to 20 carbon atoms (C₁₂-C₂₀), that is saturated or unsaturated (one or a plurality ethylene unsaturations), and
- Orga+ represents an organic cation chosen from the cationic form of mono-ethanolamine, hexadecylamine, N,N,N',N'-tetrakis-(hydroxy-propyl-2)ethylene di-amine or tris-hydroxymethyl aminomethane, alanine or tryptophan, a quaternary ammonium N⁺R₁R₂R₃-L-CO₂H, where R1, R2, R3, identical or different, denote a linear saturated alkyl radical comprising from 1 to 12 carbon atoms and L denotes a divalent saturated linear hydrocarbon radical comprising from 2 to 6 carbon atoms.
- Inorga+ represents strontium (Sr²⁺).

When Orga+ denotes a quaternary ammonium salt, R₁, R₂, R₃ are preferably identical and denote a C₁- C₄ linear saturated alkyl radical, L denotes a C₂-C₄ saturated linear hydrocarbon diradical, and particularly preferably, R₁, R₂, and R₃, denote a methyl radical and L denotes a diradical -CH₂-.

The radical R of the structures (IV) and (V) may contain one or a plurality of ethylene unsaturations.

Preferentially, R denotes a saturated or unsaturated, linear or branched hydrocarbon radical, having from 12 to 18 carbon atoms (C₁₂-C₁₈), and optionally containing one or a plurality of ethylene unsaturations.

Particularly preferably, R denotes a C₁₂-C₁₈ linear hydrocarbon radical, said radical optionally containing 1, 2 or 3 ethylene unsaturations,

Of the novel compounds having formula (IV) or (V), mention may be made of the following compounds (A'), (B'), (C'), (D'), (A"), (B"), (C") (D") and mixtures thereof: 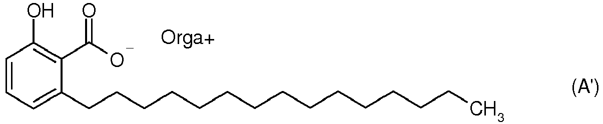 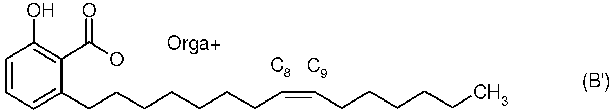 Or 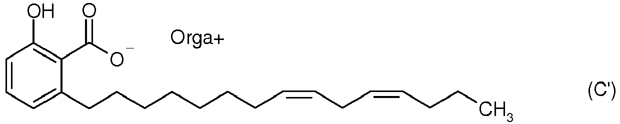 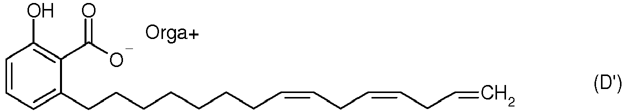 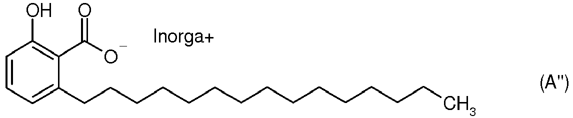 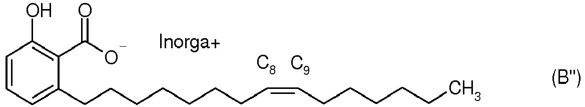 Or 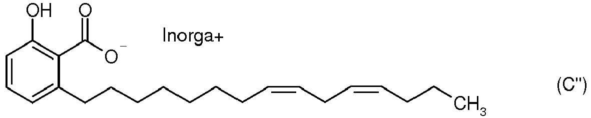 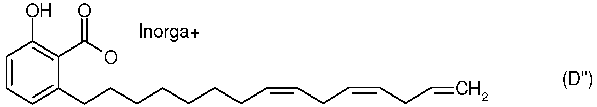 where Orga + and Inorga+ have the same meaning as described above.

### c) Preparation of compounds having formula (II)

The compounds having formula (II) according to the invention may be obtained by means of a salification reaction of the corresponding acid compounds having formula (I).

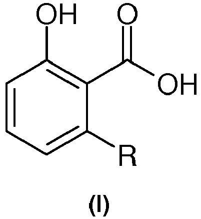

The compounds having formula (I) may be prepared using methods known to those skilled in the art. More particularly, the methods described hereinafter given by way of example may be used.

The compounds having formula (I) may be obtained in 2 or 3 steps from compounds A2 and B1 with reference to the diagram hereinafter. The compound A2 (x = 1-9) may be obtained by means of synthesis in 8 steps as described in Chem. Pharm. Bull. 2001, 49, 18-22 from compound A1.

The compound B1 may be obtained in four steps from 3-butynol as described in Chem. Pharm. Bull. 2001, 49, 18-22.

The compound B2 may be obtained by Wittig coupling between compounds A2 and B1 in the presence of butyl lithium in a non-polar solvent such as hexane for example, at ambient temperature, in an inert atmosphere.

The compound C may be obtained from compound B2 by reacting with BBr3 in dichloromethane for example followed by treatment with soda in a protic solvent such as ethanol. 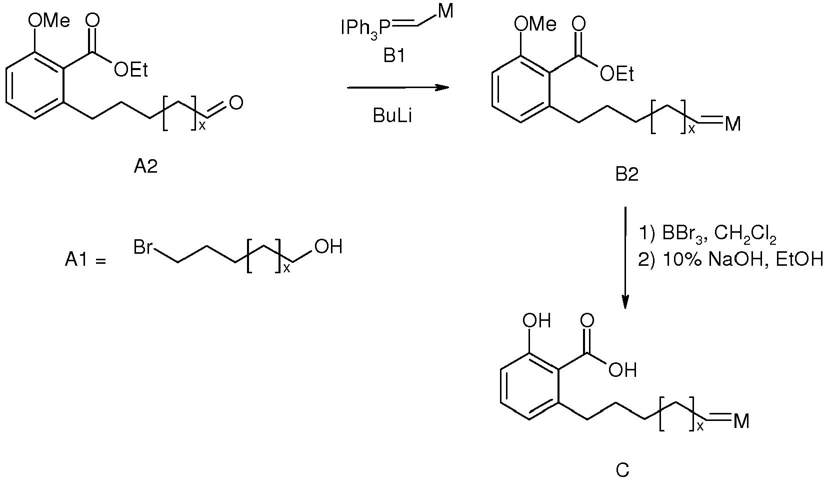 where M denotes a saturated C₆-C₁₄ hydrocarbon radical, such as for example C₆H₁₃, or mono-unsaturated CₙH₂ₙ₋₁ where n varies from 6 to 14 such that -CH₂-CH=CH-C₅H₁₁, or -CH₂-CH=CH-C₃H₇, or a hydrocarbon radical comprising two ethylene unsaturations (di-unsaturated) CₘH₂ₘ₋₃ where m varies from 6 to 14, such that -CH₂-CH=CH-CH₂-CH=CH₂.

According to the nature of the radical M, the compound C obtained comprises a hydrocarbon chain containing 1, 2 or 3 unsaturations (C corresponds to the mono, di or tri-unsaturated compounds having formula (I)).

In order to obtain the corresponding saturated compounds having formula (I), a saturated radical M is preferentially used and the double bond introduced during the Wittig reaction is reduced by catalytic hydrogenation, for example with Pd/C in a protic solvent, this hydrogenation optionally being carried out either on compound B2 or after the reaction with BBr3/NaOH, on compound C.

The compounds having formula (II) are obtained by means of salification of the compounds having formula (I) obtained, according to the protocol described hereinafter in the specific case of the anacardic acids mixture.

In one preferred alternative embodiment, the compounds having formula (II) in a mixture may also be obtained from a raw material of natural origin. As such, the mixture of compounds having formula (II) for which R denotes a C15 linear hydrocarbon chain may be obtained in 2 steps from the anacardic acid mixture.

In one preferred alternative embodiment, the compounds having formula (II) in a mixture may also be obtained from a raw material of natural origin. As such, the mixture of compounds having formula (II) wherein R denotes a C15 linear hydrocarbon chain may be obtained in 2 steps from anacardic acid.

Anacardic acid is a mixture of four 6-alkylsalicylic acid compounds: 6-pentadecylsalicylic acid (A), 6-[8(Z)-pentadecenyl]salicylic acid (B), 6-[8(Z),-11(Z)-pentadecadienyl]salicylic acid (C), and 6-[8(Z),11(Z),-14-pentadecatrienyl]salicylic acid (D) (RN: 11034-77-8 for mixture (A), (B), (C), (D); RN: 16611-84-0 for 6-pentadecylsalicylic acid (A)).

In a first step, the anacardic acid mixture RN: 11034-77-8 may be obtained from "raw" or "natural" material called CNSL ("Cashew Nut Shell Liquid"), extracted from cashew nut shell (*Anacardium occidentale*), generally containing 60 to 80% anacardic acid.

This mixture comprises the following compounds:

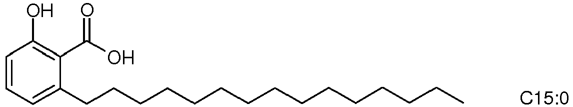

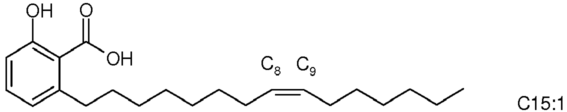

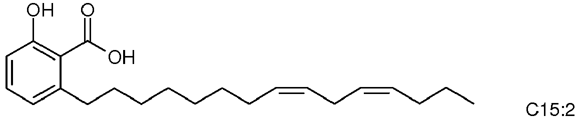

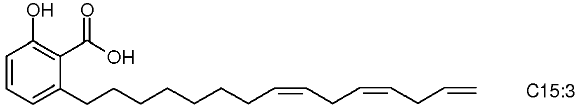

It consists of a "mixture C15" of natural origin.

"Mixture C15" may be isolated from natural CNSL according to the method described in J. Agric. Food Chem. 2001, 49, 2548-2551:

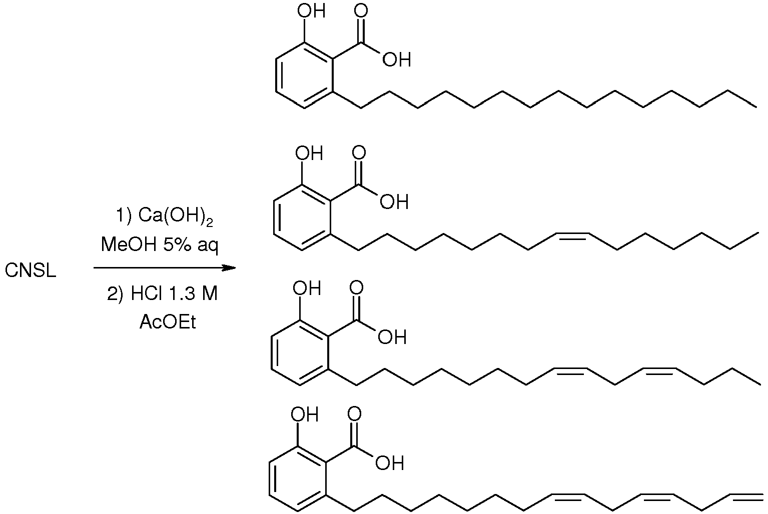

### Salification:

The preparation of the sodium salt of "mixture C15" (anacardic acid, RN: 11034-77-8) is described in J. Am. Mosquito Control Association 2009, 25, 386-389 and in Proceedings of the Institution of Chemists 1961, 81-85.

The salts having formula (II) may be obtained by adding 1 equivalent of the salifying compound to the compound (I), or to the mixture of compounds (I).

Particularly, the salts having formula (II) may be obtained by adding 1 equivalent of the salifying compound in aqueous solution form to a solution in an alcohol such as isopropanol of the compound (I), or the mixture of compounds (I). The salts having formula (II) are isolated by evaporating the solvent.

The sodium or lysine salts may be obtained for example by adding aqueous solutions, respectively of soda or lysine (one equivalent), to the acid compound alone or in a mixture dissolved in a solvent such as methanol followed by evaporating the solvent.

The invention further describes compositions comprising in a physiologically acceptable medium at least one compound or mixture of compounds having formula (IV) or (V) or any of the tautomeric forms thereof, any of the optical isomers thereof, any of the geometric isomers thereof or any of the solvates thereof such as hydrates.

The invention particularly relates to a composition, particularly a cosmetic composition, comprising in a physiologically acceptable medium:
i) a mixture of compounds having formula (II) as defined above or any of the tautomeric forms thereof, any of the optical isomers thereof, any of the geometric isomers thereof or any of the solvates thereof such as hydrates and ii) at least one antiperspirant agent and/or at least one further deodorant agent (additional deodorant) in a physiologically acceptable medium.

### Cosmetic composition

The composition according to the invention may contain one or a plurality of additional deodorant agents other than those having formula (II), and more particularly other than those having formula (IV) or (V).

### Additional deodorant agents

Of the additional deodorant agents other than the compounds according to the invention, mention may be made for example of:
- Bacteriostatic agents or other bactericidal agents such as 2,4,4'-trichloro-2'-hydroxydiphenylether (Triclosan), 2,4-dichloro-2'-hydroxydiphenylether, 3',4',5'-trichlorosalicylanilide, 1-(-3',4'-dichlorophenyl)-3-(4'chlorophenyl)urea (Triclocarban) or 3,7,11-trimethyldodeca-2,5,10-trienol (Farnesol); quaternary ammonium salts such as cetyltrimethylammonium salts, cetylpyridinium salts; chlorhexidine and salts; diglycerol monocaprate, diglycerol monolaurate, glycerol monolaurate; polyhexamethylene biguanide salts;
- zinc salts such as zinc salicylate, zinc phenolsulfonate, zinc pyrrolidone carboxylate (more commonly referred to as zinc pidolate), zinc sulfate, zinc chloride, zinc lactate, zinc gluconate, zinc ricinoleate, zinc glycinate, zinc carbonate, zinc citrate, zinc chloride, zinc laurate, zinc oleate, zinc orthophosphate, zinc stearate, zinc tartrate, zinc acetate or mixtures thereof;
- odor absorbers such as zeolites, cyclodextrins, metal oxide silicates such as those described in the application US2005/063928; metal oxide particles modified by a transition metal such as those described in the applications US2005084464 and US2005084474, aluminosilicates such as those described in the application EP1658863, chitosan derivative particles such as those described in the patent US6916465;
- substances inhibiting the enzyme reactions responsible for the formation of odorous compounds such as arylsulfatase, 5-Lipoxygenase, aminocyclase, β-glucoronidase inhibitors;
   and mixtures thereof.

The additional deodorant agents may be present in the composition according to the invention at a rate of 0.01 to 20% by mass in relation to the total mass of the composition, and preferably at a rate of 0.1 to 10% by mass.

The compositions according to the invention may contain one or a plurality of antiperspirant agents other than those having formula (II), and more particularly other than those having formula (IV) or (V).

### Antiperspirant agents

The antiperspirant agents are preferably chosen from aluminum and/or zirconium salts; zirconium hydroxychloride and aluminum hydroxychloride complexes with an amino acid such as those described in the patent US-3792068. Such complexes are generally known under the name ZAG (when the amino acid is Glycine). ZAG complexes generally have an Al/Zr quotient ranging from approximately 1.67 to 12.5 and a Metal/CI quotient ranging from 0.73 to 1.93. Of these products, mention may be made of aluminum zirconium octachlorohydrex GLY, aluminum zirconium pentachlorohydrex GLY, aluminum zirconium tetrachlorohydrate GLY and aluminum zirconium trichlorohydrate-GLY.

Of the aluminum salts, mention may be made of aluminum chlorohydrate, aluminum chlorohydrex, aluminum chlorohydrex PEG, aluminum chlorohydrex PG, aluminum dichlorohydrate, aluminum dichlorohydrex PEG, aluminum dichlorohydrex PG, aluminum sesquichlorohydrate, aluminum sesquichlorohydrex PEG, aluminum sesquichlorohydrex PG, alum salts, aluminum sulfate, aluminum zirconium octachlorohydrate, aluminum zirconium pentachlorohydrate, aluminum zirconium tetrachlorohydrate, aluminum zirconium trichlorohydrate and more particularly aluminum hydroxychloride marketed by REHEIS as REACH 301® or by GUILINI CHEMIE as ALOXICOLL PF 40®. Aluminum and zirconium salts are for example that marketed by REHEIS as REACH AZP-908-SUF®.

Aluminum chlorohydrate in activated or non-activated form shall more particularly be used.

The antiperspirant agents may be present in the composition according to the invention at a rate of 0.001 to 30% by mass in relation to the total mass of the composition, and preferably at a rate of 0.5 to 25% by mass.

### Dosage forms

The composition according to the invention may be presented in any of the dosage forms conventionally used for topical application and particularly in the form of aqueous gels, aqueous or hydroalcoholic solutions. They may also, by adding a fatty or oil phase, be presented in the form of dispersions such as lotion, emulsions of liquid or semi-liquid consistency such as milk, obtained by dispersing a fatty phase in an aqueous phase (O/W) or conversely (W/O), or suspensions or emulsions of soft, semi-solid or solid consistency such as cream or gel, or multiple emulsions (W/O/W or O/W/O), microemulsions, ionic and/or non-ionic type vesicle dispersions, or wax/aqueous phase dispersions. These compositions are prepared according to routine methods.

The compositions may be particularly packaged in pressurized form in an aerosol device or in a pump bottle; packaged in a device equipped with a perforated wall particularly a grid; packaged in a device equipped with a roll-on applicator; packaged in stick form, in loose or compact powder form. In this respect, they contain the ingredients generally used in this type of products and well-known to those skilled in the art.

According to a further specific form of the invention, the compositions according to the invention may be anhydrous.

The term anhydrous composition denotes a composition containing less than 2% by mass of water, or less than 0.5% of water, and particularly free from water, water not being added during the preparation of the composition but corresponding to the residual water provided by the mixed ingredients.

According to a further specific form of the invention, the compositions according to the invention may be solid particularly in stick form.

The term "solid composition" denotes that the measurement of the maximum force measured by means of texturometric analysis on inserting a probe in the sample of formula should be at least equal to 0.25 Newton, in particular at least equal to 0.30 Newton, particularly at least equal to 0.35 Newton, assessed under precise measurement conditions as follows.

The formulas are poured when heated into jars 4 cm in diameter and 3 cm at the bottom. Cooling is performed at ambient temperature. The hardness of the formulas produced is measured after waiting 24 hours. The jars containing the samples are characterized by means of texturometric analysis using a texturometer such as that marketed by Rheo TA-XT2, according to the following protocol: a 5 mm diameter stainless steel ball type probe is brought into contact with the sample at a speed of 1 mm/s. The measurement system detects the interface with the sample with a detection threshold equal to 0.005 Newtons. The probe is inserted 0.3 mm into the sample, at a rate of 0.1 mm/s. The measurement apparatus records the progression of the compression force measured over time, during the penetration phase. The hardness of the sample corresponds to the mean of the maximum values of the force detected during penetration, for at least 3 measurements.

### Aqueous phase

The compositions according to the invention intended for cosmetic use may comprise at least one aqueous phase. They are particularly formulated in aqueous lotions or in water-in-oil, oil-in-water emulsion, or in multiple emulsion (triple oil-in-water-in-oil or water-in-oil-in-water) (such emulsions are known and described for example by C. FOX in "Cosmetics and Toiletries" - November 1986 - Vol 101 - pages 101-112).

The aqueous phase of said compositions contains water and generally further water-soluble or miscible solvents. The water-soluble or miscible solvents comprise short-chain mono-alcohols for example C1-C4 such as ethanol, isopropanol; diols or polyols such as ethyleneglycol, 1,2-propyleneglycol, 1,3-butylene glycol, hexyleneglycol, diethyleneglycol, dipropylene glycol, 2-ethoxyethanol, diethylene glycol monomethylether, triethylene glycol monomethylether and sorbitol. Propyleneglycol and glycerin, propane 1,3 diol shall more particularly be used.

The composition according to the invention preferably has a pH ranging from 3 to 9 according to the substrate chosen.

When the composition is in the form of emulsion, it generally contains according to the nature of the emulsion one or a plurality of emulsifying surfactants.

The total quantity of emulsifiers shall be preferably in the composition according to the invention at active substance concentrations ranging from 1 to 8% by mass and more particularly from 2 to 6% by mass in relation to the total mass of the composition.

### Fatty phase

The compositions according to the invention may contain at least one non-water-miscible organic liquid phase, known as a fatty phase. This generally includes one or a plurality of hydrophobic compounds rendering said phase non-miscible in water. Said phase is liquid (in the absence of a structuring agent) at ambient temperature (20-25°C). Preferentially, the non-water-miscible organic liquid phase according to the invention generally comprises at least one volatile oil and/or a non-volatile oil and optionally at least one structuring agent.

The term "oil" denotes a liquid fat at ambient temperature (25°C) and atmospheric pressure (760mm Hg i.e. 1.05x10⁵ Pa). The oil may be volatile or non-volatile.

The term "volatile oil" according to the invention denotes any oil capable of evaporating on contact with skin or keratin fiber, in less than one hour, at ambient temperature and at atmospheric pressure. The volatile oils according to the invention are volatile cosmetic oils, which are liquid at ambient temperature, having a vapor pressure different to zero, at ambient temperature and atmospheric pressure, particularly ranging from 0.13 Pa to 40,000 Pa (10⁻³ at 300 mm Hg), particularly ranging from 1.3 Pa to 13,000 Pa (0.01 to 100 mm Hg), and more specifically ranging from 1.3 Pa to 1300 Pa (0.01 at 10 mm Hg).

The term "non-volatile oil" denotes an oil remaining on skin or keratin fiber at ambient temperature and atmospheric pressure for at least several hours and particularly having a vapor pressure less than 10⁻³ mm Hg (0.13 Pa).

The oil may be chosen from any physiologically acceptable and particularly cosmetically acceptable oils, in particular mineral, animal, plant, synthetic oils; in particular, volatile or non-volatile hydrocarbon and/or silicone and/or fluorinated oils and mixtures thereof.

More specifically, the term "hydrocarbon oil" denotes an oil essentially comprising carbon and hydrogen atoms and optionally one or a plurality of functions chosen from hydroxyl, ester, ether, carboxylic functions. Generally, the oil has a viscosity of 0.5 to 100,000 mPa.s, preferably from 50 to 50,000 mPa.s and more preferably from 100 to 300,000 mPa.s.

By way of examples of volatile oils suitable for use in the invention, mention may be made of
- volatile hydrocarbon oils chosen from hydrocarbon oils having 8 to 16 carbon atoms, and particularly petroleum-based C₈-C₁₆ isoalkanes (also referred to as isoparaffins) such as isododecane (also referred to as 2,2,4,4,6-pentamethylheptane), isodecane, isohexadecane, and for example the oils sold under the trade names Isopars or Permetyls, C₈-C₁₆ branched esters, iso-hexyl neopentanoate, and mixtures thereof.
- volatile silicones, such as for example volatile linear or cyclic silicone oils, particularly those having a viscosity ≤ 8 centistokes (8 10⁻⁶ m²/s), and having in particular 2 to 7 silicon atoms, these silicones optionally comprising alkyl or alkoxy groups having 1 to 10 carbon atoms, and mixtures thereof.

Mention may be made, as a volatile silicone oil suitable for use in the invention, in particular, of octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, dodecamethyl pentasiloxane, and mixtures thereof.
- plant-based hydrocarbon oils such as liquid fatty acid triglycerides having 4 to 24 carbon atoms such as heptanoic or octanoic triglycerides or wheat germ, olive oils, sweet almond, palm, rapeseed, cotton, alfalfa, poppy seed, pumpkin, squash, blackcurrant seed, evening primrose, millet, barley, quinoa, rye, safflower, candlenut, passiflora, musk rose, sunflower, corn, soybean, squash, grape seed, sesame, hazelnut, apricot, macadamia, castor, avocado oils, caprylic/capric acid triglycerides such as those sold by Stearineries Dubois or those sold under the trade names Miglyol 810, 812 and 818 by Dynamit Nobel, jojoba oil;
- linear or branched hydrocarbons of mineral or synthetic origin, such as liquid paraffins and derivatives thereof, petroleum jelly, polydecenes, polybutenes, hydrogenated polyisobutene such as Parleam, squalane;
- synthetic ethers having 10 to 40 carbon atoms such as dicaprylether;
- synthetic esters particularly of fatty acids such as the oils having the formula R¹COOR² wherein R¹ represents the residue of a linear or branched higher fatty acid comprising 1 to 40 carbon atoms and R² represents a hydrocarbon chain, particularly branched containing 1 to 40 carbon atoms where R¹ + R² ≥ 10 such as isononyl isononanoate, isopropyl myristate, isopropyl palmitate, C₁₂ to C₁₅ alcohol benzoate,
- liquid fatty alcohols at ambient temperature with a branched and/or unsaturated carbon chain having 12 to 26 carbon atoms such as octyl dodecanol;
- silicone oils, such as polydimethylsiloxanes (PDMS) which are non-volatile, linear (dimethicone) or cyclic (cyclomethicones); polydimethylsiloxanes comprising alkyl, alkoxy or phenyl groups which are pendant or at the end of the silicone chain, said groups having 2 to 24 carbon atoms; phenylated silicones, such as phenyl trimethicones, phenyl dimethicones, phenyltrimethylsiloxydiphenylsiloxanes, diphenyl dimethicones, diphenylmethyldiphenyl-trisiloxanes or (2-phenylethyl)trimethylsiloxysilicates, and
- mixtures thereof.

### Additives

The cosmetic compositions according to the invention may further comprise cosmetic adjuvants chosen from softeners, antioxidants, opacifiers, stabilizers, hydrating agents, vitamins, bactericides, preservatives, polymers, perfumes, a fatty phase structuring agent particularly chosen from waxes, pasty compounds, gelling agents; organic or inorganic fillers; thickening or suspension agents, propellants or any other ingredient routinely used in cosmetics for this type of application.

Obviously, those skilled in the art shall take care to choose the optional compound(s) in such a way that the advantageous properties intrinsically associated with the cosmetic composition according to the invention are not, or are practically not, altered by the envisaged addition(s).

### Aerosols

The compositions according to the invention may be pressurized and packaged in an aerosol device consisting of:
(A) at least one container comprising a composition according to the invention,
(B) at least one propellant and at least one means for dispensing said composition in aerosol form.

The propellants generally used in this type of product and well-known to those skilled in the art, such as for example dimethylether (DME); volatile hydrocarbons such as n-butane, propane, isobutane, and mixtures thereof, optionally with at least one chlorinated and/or fluorinated hydrocarbon; of the latter, mention may be made of the compounds sold by Dupont de Nemours under the trade names Freon® and Dymel®, and in particular monofluorotrichloromethane, difluorodichloromethane, tetrafluorodichloroethane and 1,1-difluoroethane particularly sold under the trade name DYMEL 152 A® by DUPONT. Carbon dioxide, nitrous oxide, nitrogen or compressed air may also be used as a propellant.

The compositions containing perlite particles as defined above and the propellant(s) may be located in the same compartment or in different compartments in the aerosol container. According to the invention, the propellant concentration generally varies from 5 to 95% by pressurized mass and more preferentially from 50 to 85% by mass in relation to the total mass of the pressurized composition.

The dispensing means, forming part of the aerosol device, generally consist of a dispensing valve controlled by a dispensing head, in turn comprising a nozzle via which the aerosol composition is sprayed. The container containing the pressurized composition may be opaque or transparent. It may be made of glass, polymeric or metal material, optionally coated with a layer of protective varnish.

The expressions "between ... and ..." and "ranging from ... to ..." are to be understood to be inclusive of the limits, unless specified otherwise.

The following examples illustrate the present invention without limiting the scope thereof.

In the examples, the temperature is ambient (20-25°C), the pressure atmospheric (101,325 Pa), unless specified otherwise. The mass of the ingredients is expressed as a percentage in relation to the mass of the total composition in question.

### Examples

### Example 1: Synthesis

### Example 1.1: Preparation of lysine salt mixture M1

Mixture M1 consisting of the following salified compounds 5, 6, 7 and 8 was prepared: 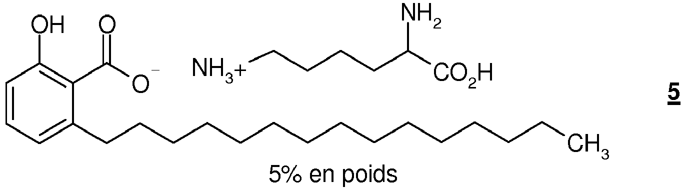 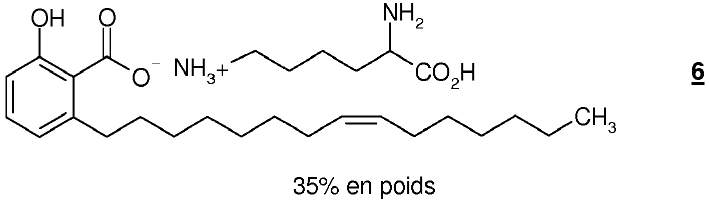 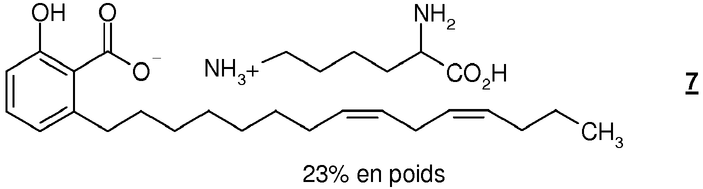 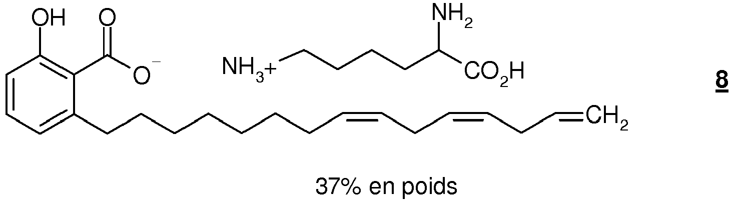 32.82 g (1 equivalent) of L-lysine (Aldrich reference L5501) in 150 mL of water is added slowly to 80 g of mixture **M0** of the following compounds: 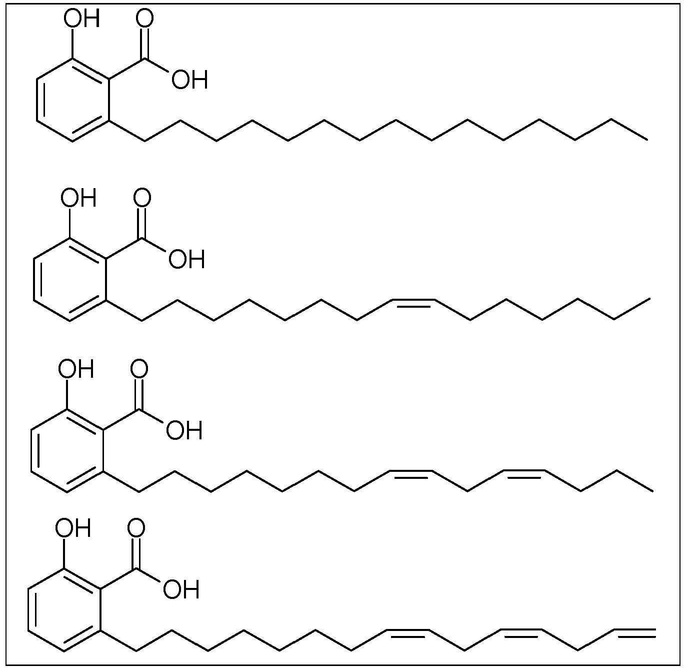 (mean molar mass = 356 g/mol) solubilized in 500 mL of MeOH at a temperature of 10/15°C. At the end of the addition, pH=7. The solution is evaporated to dryness to result in a beige powder.

Analyses: NMR, elemental analysis confirming that the expected compound was obtained.

### Example 1.2: Preparation of sodium salt mixture M2:

Mixture M2 consisting of the following salified compounds 1, 2, 3 and 4 was prepared: 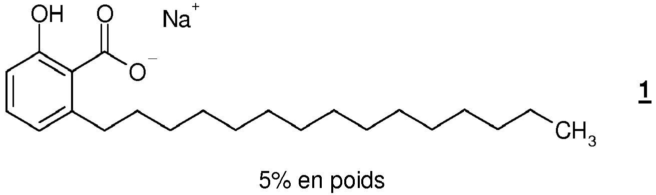 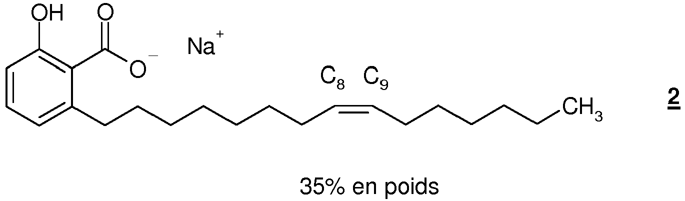 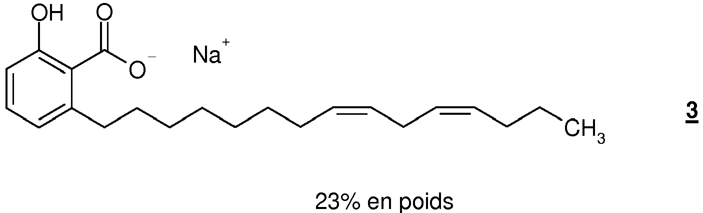 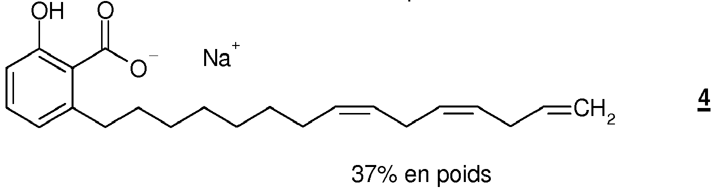 8.98 g (1 equivalent) of soda (NaOH) in 100 mL of water is added slowly to 80 g of mixture M**0** of the following compounds: 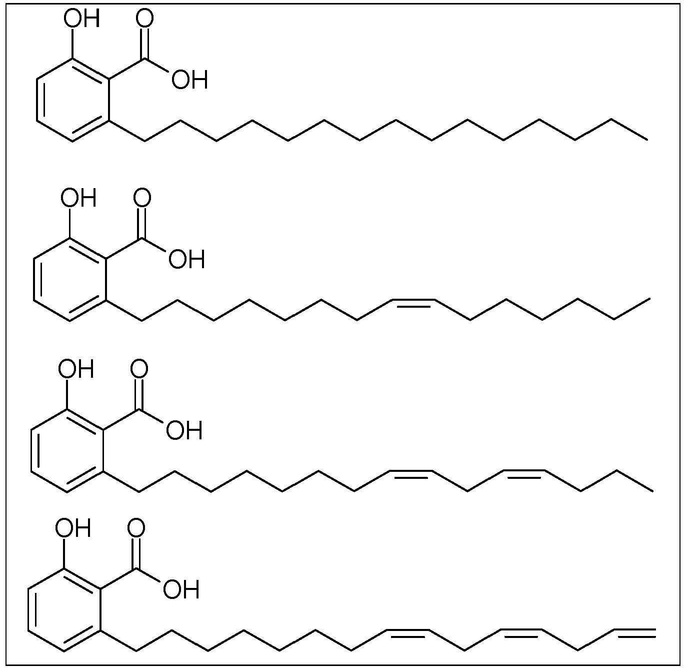 (mean molar mass = 356 g/mol) solubilized in 500 mL of MeOH at a temperature of 10/15°C. At the end of addition, pH=7.4. The solution is evaporated to dryness under reduced pressure to result in a beige powder.

The analyses confirmed that the expected mixture M2 was obtained.

### Example 2: Effect on Corynebacterium xerosis:

- **Test principle:** contacting of decreasing concentrations of compounds under evaluation with an identical inoculum of microorganisms in a culture medium suitable for the growth of *C. xerosis.* After incubating the microplate for 24 to 48 hours according to the strains, the Optical Density (620 nm) is measured and the results are expressed as a percentage of growth calculated in relation to a growth control free from the compound under evaluation.
- **Definition of MIC (minimum inhibitory concentration):** the lowest concentration of compound under evaluation limiting growth significantly compared to the control is considered to be inhibitory.

The dilutions of the compounds under evaluation are performed in agar at 1:1000, making it possible, for dispersible compounds under evaluation, to do away with the use of a solvent which could introduce bias in the evaluation of the compounds. The concentration range chosen was: 1%, 0.3%, 0.03% and 0.003%. Each concentration was tested in triplicate.

To ensure that the potential inhibitory activity was not associated with a pH effect, the pH of the culture medium was verified at the end of incubation.

The compound mixtures M**0** and M**2** were evaluated based on this protocol, along with n-octanoyl-5-salicylic acid (comparison).

The mixture M**0** is the mixture of anacardic acids (comparison) ("mixture C15") described above.

The mixture M**2** is the "sodium salt mixture C15" (invention) as per example 1.2

It was observed that mixtures M**0** and M**2** and the compound n-octanoyl-5-salicylic acid inhibit the growth of *C. xerosis* but the mixture M**2** having formula (II) is much more effective on C xerosis growth than the mixture M**0** having formula (I) as indicated in table 1.

**Table 1**

| **Compound mixture** | **MIC *C. xerosis*** | **final pH of culture medium** |
|---|---|---|
| Mixture M**0** | 0.03% | 7 |
| Mixture M2 | < 0.003% | 7 |
| N-octanoyl-5-salicylic acid (MEXORYL SAB®-CHIMEX) | < 0.003% | 7 |

### Example 3: Anti-odor effect (on incubated sweat):

The deodorant activity was evaluated in the *ex-vivo* olfactory evaluation test, on incubated sweat.

Each compound under evaluation was introduced at a rate of 0.3 mg into 1 mL of sweat. The compounds under evaluation were weighed in 25 or 30 mL pill bottles and 1 mL from a pool of fresh sweat was then added. The pill bottles were hermetically sealed and placed in an incubator at 35°C for 24 hours. After being removed from the incubator, the pill bottles were opened and allowed to cool to ambient temperature.

A panel of sixteen people performed an olfactory analysis of each pill bottle. The efficacy of each compound evaluated was determined in comparison to a negative control (incubated sweat alone).

**Table 2**

| Mixture under test | **% Variation incubated sweat intensity** |
|---|---|
| Mixture M**0** | -45 |
| Mixture M2 | -90 |
| N-octanoyl-5-salicylic acid (MEXORYL SAB®- CHIMEX) | -57 |
| Incubated sweat alone (control) | 0 |

It was observed that the mixtures M**0** and M**2** and the compound n-octanoyl-5-salicylic acid reduce the unpleasant odor of incubated sweat and that the compound M**2** makes it possible to reduce the odor of sweat much more significantly than the mixture M**0** or the compound n-octanoyl-5-salicylic acid.

### Example 4: Formulations

### Example 4.1: Deodorant formulation

| | |
|---|---|
| Carbopol ® | 0 |
| Preservatives | 1 |
| Mixture M2 | 0.1% |
| Water | qs 100 |

(% mass in relation to mass of total composition).

The composition produces a deodorant effect.

### Example 4.2: Deodorant formulation

| | |
|---|---|
| Carbopol ® | 0 |
| Preservatives | 1 |
| Mixture M2 | 0.3% |
| Water | qs 100 |

(% mass in relation to mass of total composition)

The composition produces a deodorant effect.

### Example 4.3: Deodorant and antiperspirant formulation

| | |
|---|---|
| Carbopol ® | 0 |
| Preservatives | 1 |
| Aluminum chlorhydroxide 50% | 18% |
| Aluminum chloride hexahydrate 50% | 6% |
| Mixture M2 | 0.1% |
| Water | qs 100 |

(% mass in relation to mass of total composition)

The composition produces a deodorant and antiperspirant effect.

### Example 4.4: Deodorant and antiperspirant formulation

| | |
|---|---|
| Carbopol ® | 0 |
| Preservatives | 1 |
| Aluminum chlorhydroxide 50% | 15% |
| Aluminum chloride hexahydrate 50% | 8% |
| Compound M2 | 0.3% |
| Water | qs 100 |

(% mass in relation to mass of total composition)

The composition produces a deodorant and antiperspirant effect.

## Claims

1. Use as a deodorant agent of a mixture of compounds having formula (II) for treating body odor, particularly underarm odor: 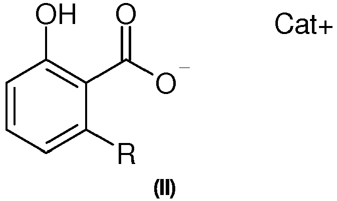
where R denotes a saturated or unsaturated linear or branched hydrocarbon radical comprising from 12 to 20 carbon atoms, where said radical R may comprise one or a plurality of ethylene unsaturations (of the double bond type) and
Cat+ represents a cation or a mixture of cations, organic or inorganic, suitable for obtaining electroneutrality of the compound or mixture of compounds having formula (II),
**characterized in that** a mixture of at least 4 compounds having formula (II), denoted compounds **(IIA), (IIB) (IIC), (IID),** is used, wherein:
- the radicals R of each of the compounds **(IIA), (IIB), (IIC), (IID)** denote a linear hydrocarbon radical comprising from 12 to 18 carbon atoms, and preferably having the same number of carbon atoms for each of the compounds **(IIA), (IIB), (IIC), (IID):**
- the compound **(IIA),** having a saturated radical R; and
- the compound **(IIB),** having a mono-unsaturated radical R; and
- the compound **(IIC),** having a di-unsaturated radical R; and
- the compound **(IID),** having a tri-unsaturated radical R; and
- Cat+ having the same meaning for each of the compounds **(IIA), (IIB) (IIC), (IID).**

2. Use according to claim 1, **characterized in that** R denotes a C₁₄-C₁₆ linear hydrocarbon radical.

3. Use according to claim 1 or 2, **characterized in that**
- the inorganic cation Cat+ is chosen from alkaline metals, alkaline-earth metals, transition metals;
- the organic cation Cat+ is chosen from the cationic form of an amine or a quaternary ammonium.

4. Use according to any of claims 1 to 3, **characterized in that** the organic Cat+ is the cationic form of an L or D form amino acid or a quaternary ammonium N⁺R₁R₂R₃-L-CO₂H, where R₁, R₂, R₃, identical or different, denote a linear saturated alkyl radical comprising 1 to 12 carbon atoms and L denotes a divalent saturated hydrocarbon radical comprising 2 to 6 carbon atoms.

5. Use according to claim 3, **characterized in that** Cat+ represents Na+.

6. Use according to claim 3, **characterized in that** Cat+ is the cationic form of lysine.

7. Use according to any of claims 1 to 6, **characterized in that** a mixture of the compounds **(IIA), (IIB), (IIC), (IID)** is used, wherein:
- the proportion of compound **(IIA),** varies from 0.001 to 5%,
- the proportion of compound **(IIB)** varies from 20 to 45%,
- the proportion of compound **(IIC)** varies from 5 to 35%,
- the proportion of compound **(IID)** varies from 25 to 50%, the percentages being by mass in relation to the total mass of the mixture of compounds **(IIA), (IIB) (IIC)** and **(IID)**.

8. Use according to any of claims 1 to 7 , **characterized in that** a mixture of the compounds **(IIA), (IIB), (IIC), (IID)** is used, wherein R denotes a hydrocarbon radical having 15 carbon atoms.

9. Use according to any of claims 1 to 8, **characterized in that** the compounds **(IIA), (IIB) (IIC), (IID),** are chosen from: 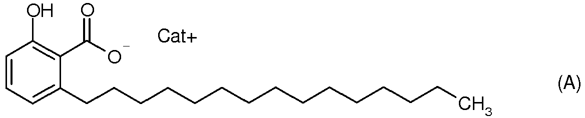 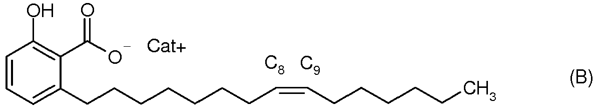 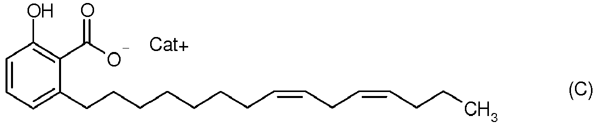 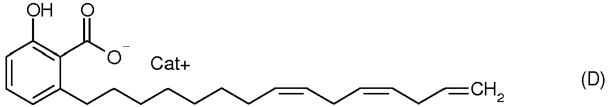 and more particularly compounds **(IIA), (IIB) (IIC), (IID)** are a mixture wherein:
- the proportion of compound (A) varies from 0.001 to 5%,
- the proportion of compound (B) varies from 20 to 45%,
- the proportion of compound (C) varies from 5 to 35%,
- the proportion of compound (D) varies from 25 to 50%, the percentages being by mass in relation to the total mass of the mixture of compounds (A), (B), (C) and (D).

10. Use according to any of claims 1 to 9, **characterized in that** the compounds **(IIA), (IIB) (IIC), (IID),** are chosen from the following compounds **1, 2, 3, 4, 5, 6, 7, 8:** 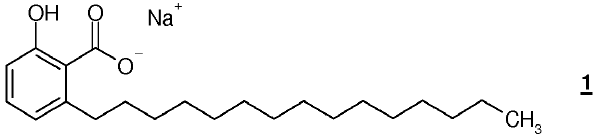 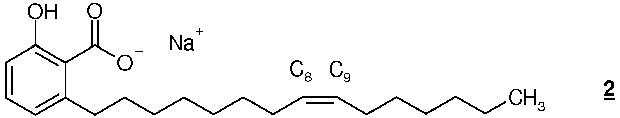 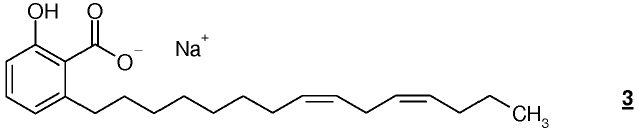 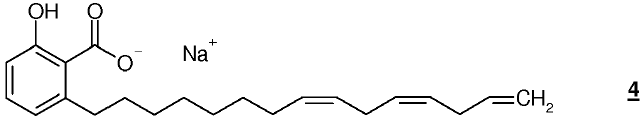 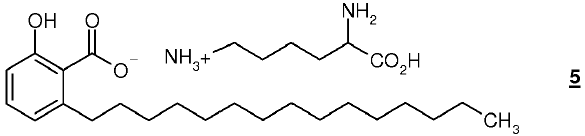 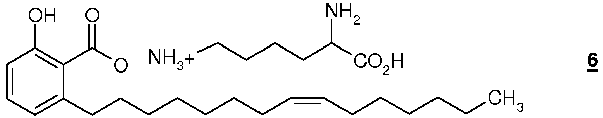 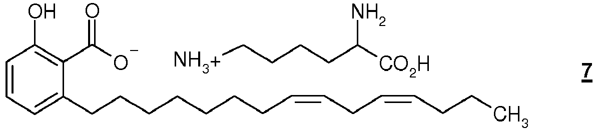 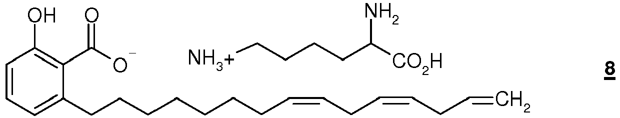 and more preferentially the compounds **(IIA), (IIB) (IIC), (IID)** are a mixture of the compounds **1, 2, 3** and **4** or a mixture of the compounds **5, 6, 7** and **8,** and even more preferentially a mixture wherein the **compounds 1, 2, 3, 4** or the compounds **5, 6, 7 and 8** are in the same proportions as the compounds (A), (B), (C) and (D) according to claim 7, respectively.

11. Use according to claim 10, **characterized in that** the compounds (**IIA**), (**IIB**) **(IIC), (IID),** are chosen from the following mixtures:
(i) a mixture comprising 5% by mass of compound **1,** 35% of compound **2,** 23% by mass of compound **3** and 37% of compound **4;**
(ii) a mixture comprising 5% by mass of compound **5,** 35% of compound **6,** 23% by mass of compound **7** and 37% of compound **8.**

12. Composition comprising in a physiologically acceptable medium:
i) a mixture of compounds having formula (II) or any of the tautomeric forms thereof, the optical isomers thereof, the geometric isomers thereof or the solvates thereof, as defined according to any of claims 1 to 11,
ii) at least one antiperspirant agent and/or at least one further deodorant agent.

13. Aerosol device consisting of:
(A) at least one container comprising a composition as defined according to claim 12.
(B) at least one propellant and at least one means for dispensing said composition in aerosol form.

14. Cosmetic method for treating body odor, particularly underarm odor, consisting of applying on the surface of a human keratin material a composition according to claim 12 .

## Patentansprüche

1. Verwendung einer Mischung aus Verbindungen mit der Formel (II) zum Behandeln von Körpergeruch, insbesondere Achselgeruch: 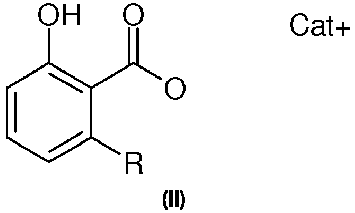
wobei R ein gesättigtes oder ungesättigtes lineares oder verzweigtes Kohlenwasserstoff-Radikal bezeichnet, welches von 12 bis 20 Kohlenstoffatome aufweist, wobei das Radikal R eine oder eine Vielzahl von Ethylen-Unsättigungen (vom Doppelbindungstyp) aufweisen kann und
Cat+ ein Kation oder eine Mischung aus Kationen, organisch oder anorganisch, darstellt, die geeignet sind zum Erhalten einer Elektroneutralität der Verbindung, oder Mischung aus Verbindungen, mit der Formel (II),
**dadurch gekennzeichnet, dass** eine Mischung von mindestens 4 Verbindungen mit der Formel (II), bezeichnet als Verbindungen **(IIA), (IIB) (IIC), (IID),** verwendet wird, wobei:
- die Radikale R von jeder der Verbindungen **(IIA), (IIB) (IIC), (IID)** ein lineares Kohlenwasserstoff-Radikal bezeichnen, welches von 12 bis 18 Kohlenstoffatome aufweist und vorzugsweise die gleiche Anzahl an Kohlenstoffatomen für jede der Verbindungen **(IIA), (IIB) (IIC), (IID)** aufweist:
- die Verbindung **(IIA)** ein gesättigtes Radikal R aufweist; und
- die Verbindung **(IIB)** ein einfach ungesättigtes Radikal R aufweist; und
- die Verbindung **(IIC)** ein zweifach ungesättigtes Radikal R aufweist; und
- die Verbindung **(IIC)** ein dreifach ungesättigtes Radikal R aufweist; und
- Cat+ dieselbe Bedeutung für jede der Verbindungen **(IIA), (IIB) (IIC), (IID)** hat.

2. Verwendung nach Anspruch 1, **dadurch gekennzeichnet, dass** R ein C₁₄-C₁₆ lineares Kohlenwasserstoff-Radikal bezeichnet.

3. Verwendung nach Anspruch 1 oder 2, **dadurch gekennzeichnet, dass**
- das anorganische Kation Cat+ ausgewählt ist aus Alkalimetallen, Erdalkalimetallen, Übergangsmetallen;
- das organische Kation Cat+ ausgewählt ist aus der kationischen Form eines Amins oder eines quaternären Ammoniums.

4. Verwendung nach einem der Ansprüche 1 bis 3, **dadurch gekennzeichnet, dass** das organische Cat+ die kationische Form einer L- oder D-Aminosäure oder eines quaternären Ammoniums N⁺R₁R₂R₃-L-CO₂H ist, wobei R₁, R₂, R₃, identisch oder unterschiedlich, ein lineares gesättigtes Alkyl-Radikal bezeichnen, das 1 bis 12 Kohlenstoffatome aufweist, und L ein zweiwertiges gesättigtes Kohlenwasserstoff-Radikal bezeichnet, das 2 bis 6 Kohlenwasserstoffatome aufweist.

5. Verwendung nach Anspruch 3, **dadurch gekennzeichnet, dass** das Cat+ Na+ darstellt.

6. Verwendung nach Anspruch 3, **dadurch gekennzeichnet, dass** Cat+ die kationische Form von Lysin ist.

7. Verwendung nach einem der Ansprüche 1 bis 6, **dadurch gekennzeichnet, dass** eine Mischung der Verbindungen **(IIA), (IIB), (IIC), (IID)** verwendet wird, wobei:
- der Anteil der Verbindung (IIA) von 0,001 bis 5 % variiert,
- der Anteil der Verbindung (IIB) von 20 bis 45 % variiert,
- der Anteil der Verbindung (IIC) von 5 bis 35 % variiert,
- der Anteil der Verbindung (IID) von 25 bis 50 % variiert, wobei die Anteile Masseanteile im Verhältnis zur Gesamtmasse der Mischung der Verbindungen (IIA), (IIB) (IIC) und **(IID)** sind.

8. Verwendung nach einem der Ansprüche 1 bis 7, **dadurch gekennzeichnet, dass** eine Mischung der Verbindungen **(IIA), (IIB), (IIC), (IID)** verwendet wird, wobei R ein 15 Kohlenstoffatome aufweisendes Kohlenwasserstoff-Radikal bezeichnet.

9. Verwendung nach einem der Ansprüche 1 bis 8, **dadurch gekennzeichnet, dass** die Verbindungen **(IIA), (IIB), (IIC), (IID)** ausgewählt sind aus: 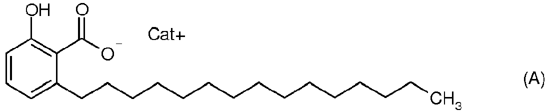 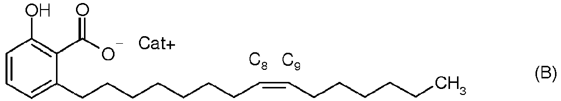 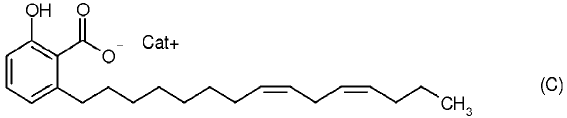 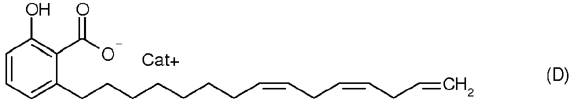 und insbesondere Verbindungen **(IIA), (IIB), (IIC), (IID)** eine Mischung sind, wobei:
- der Anteil der Verbindung (A) von 0,001 bis 5 % variiert,
- der Anteil der Verbindung (B) von 20 bis 45 % variiert,
- der Anteil der Verbindung (C) von 5 bis 35 % variiert,
- der Anteil der Verbindung (D) von 25 bis 50 % variiert, wobei die Anteile Masseanteile im Verhältnis zur Gesamtmasse der Mischung der Verbindungen (A), (B) (C) und (D) sind.

10. Verwendung nach einem der Ansprüche 1 bis 9, **dadurch gekennzeichnet, dass** die Verbindungen **(IIA), (IIB), (IIC), (IID)** aus den folgenden Verbindungen **1, 2, 3, 4, 5, 6, 7, 8** ausgewählt sind: 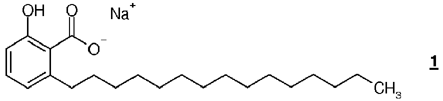 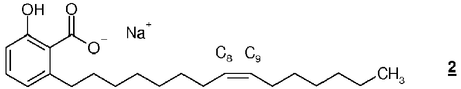 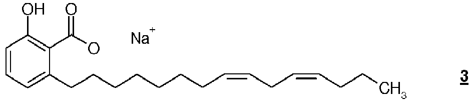 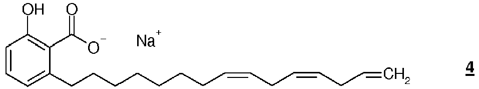 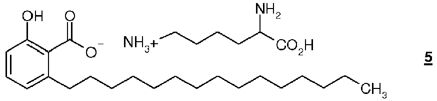 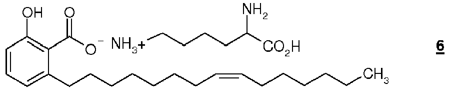 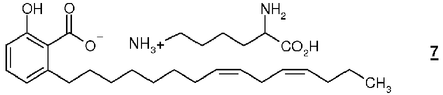 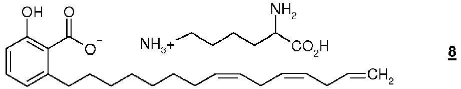 und weiter bevorzugt die Verbindungen **(IIA), (IIB) (IIC), (IID)** eine Mischung aus den Verbindungen **1, 2, 3** und 4 oder eine Mischung aus den Verbindungen **5, 6, 7** und **8 sind,** und noch weiter bevorzugt eine Mischung sind, in welcher die Verbindungen **1, 2, 3, 4** oder die Verbindungen **5, 6, 7** und **8** jeweils die gleichen Anteile aufweisen wie die Verbindungen (A), (B), (C) bzw. (D) nach Anspruch 7.

11. Verwendung nach Anspruch 10, **dadurch gekennzeichnet, dass** die Verbindungen (**IIA**), (**IIB**), (**IIC**), **(IID)** ausgewählt sind aus den folgenden Mischungen:
(i) einer Mischung aufweisend 5 Massenprozent von Verbindung **1**, 35 % von Verbindung **2**, 23 % Massenprozent von Verbindung **3** und 37 % von Verbindung **4;**
(ii) einer Mischung aufweisend 5 Massenprozent von Verbindung **5**, 35 % von Verbindung **6**, 23 % Massenprozent von Verbindung **7** und 37 % von Verbindung **8.**

12. Zusammensetzung, in einem physiologisch annehmbaren Medium enthaltend:
(i) eine Mischung aus Verbindungen mit der Formel (II) oder eine der tautomeren Formen davon, der optischen Isomere davon, der geometrischen Isomere davon oder der Solvate davon, wie nach einem der Ansprüche 1 bis 11 definiert,
(ii) mindestens ein schweißhemmendes Mittel und/oderein weiteres geruchstilgendes Mittel.

13. Aerosolvorrichtung, bestehend aus:
(A) mindestens einem Behälter, der eine nach Anspruch 12 definierte Verbindung enthält.
(B) mindestens einem Treibgas und mindestens einem Mittel zum Abgeben der Verbindung in Aerosolform.

14. Kosmetisches Verfahren zum Behandeln von Körpergeruch, insbesondere Achselgeruch, bestehend aus einem Auftragen einer Zusammensetzung nach Anspruch 12 auf eine Oberfläche eines menschlichen Keratinmaterials.

## Revendications

1. Utilisation comme agent déodorant d'un mélange de composés de formule (II) pour traiter les odeurs corporelles, en particulier les odeurs axillaires : 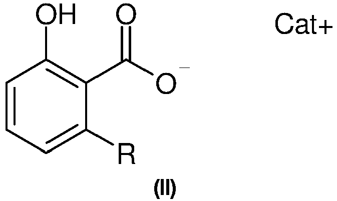
où R désigne un radical hydrocarboné linéaire ou ramifié comprenant de 12 à 20 atomes de carbone, saturé ou insaturé, ledit radical R pouvant comprendre une ou plusieurs insaturations éthyléniques (de type double liaison) et
Cat+ représente un cation ou un mélange de cations, organiques ou inorganiques, permettant d'atteindre l'électroneutralité du composé ou mélange de composés de formule (II), **caractérisée en ce qu'**on utilise un mélange d'au moins 4 composés de formule (II), désignés composés **(IIA), (IIB) (IIC), (IID),** dans lesquels :
- les radicaux R de chacun des composés **(IIA), (IIB), (IIC), (IID)** désignent un radical hydrocarboné linéaire comprenant de 12 à 18 atomes de carbone, et de préférence ayant le même nombre d'atomes de carbone pour chacun des composés **(IIA), (IIB), (IIC), (IID)** :
- le composé **(IIA),** ayant un radical R saturé ; et
- le composé **(IIB),** ayant un radical R mono-insaturé ; et
- le composé **(IIC),** ayant un radical R di-insaturé ; et
- le composé **(IID)**, ayant un radical R tri-insaturé ; et
- Cat+ ayant la même signification pour chacun des composés **(IIA), (IIB) (IIC), (IID)**.

2. Utilisation, selon la revendication 1, **caractérisée en ce que** R désigne un radical hydrocarboné linéaire en C₁₄-C₁₆.

3. Utilisation, selon la revendication 1 ou 2, **caractérisée en ce que**
- le cation Cat+ inorganique est choisi parmi les métaux alcalins, les métaux alcalino-terreux, les métaux de transition ;
- le cation Cat+ organique est choisi parmi la forme cationique d'une amine ou un ammonium quaternaire.

4. Utilisation, selon l'une quelconque des revendications 1 à 3, **caractérisée en ce que** le Cat+ organique la forme cationique d'un amino-acide de forme L ou D ou bien un ammonium quaternaire N⁺R₁R₂R₃-L-CO₂H, où R₁, R₂, R₃, identiques ou différents, désignent un radical alkyle saturé linéaire comprenant de 1 à 12 atomes de carbone et L désigne un radical hydrocarboné linéaire saturé divalent comprenant de 2 à 6 atomes de carbone.

5. Utilisation, selon la revendication 3, **caractérisée en ce que** Cat+ représente le Na+.

6. Utilisation, selon la revendication 3, **caractérisée en ce que** le Cat+ est la forme cationique de la lysine.

7. Utilisation, selon l'une quelconque des revendications 1 à 6, **caractérisée en ce qu'**on utilise un mélange des composés **(IIA), (IIB), (IIC), (IID),** dans lequel :
- la proportion de composé **(IIA),** varie de 0,001 à 5%,
- la proportion de composé **(IIB)** varie de 20 à 45%,
- la proportion de composé **(IIC)** varie de 5 à 35%,
- la proportion de composé **(IID)** varie de 25 et 50%, les pourcentages étant en masse par rapport à la masse totale du mélange des composés (**IIA**), **(IIB) (IIC)** et **(IID)**.

8. Utilisation selon l'une quelconque des revendications 1 à 7, **caractérisée en ce qu'**on utilise un mélange des composés **(IIA), (IIB), (IIC), (IID)** pour lequel R désigne un radical hydrocarboné ayant 15 atomes de carbone.

9. Utilisation selon l'une quelconque des revendications 1 à 8, **caractérisée en ce que** les composés (IIA), (IIB), (IIC) et (IID) sont choisis parmi : 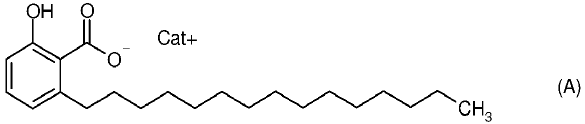 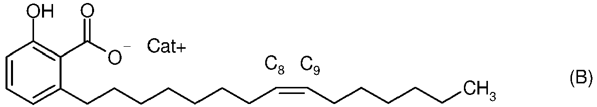 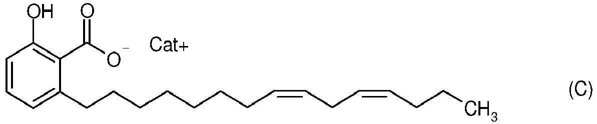 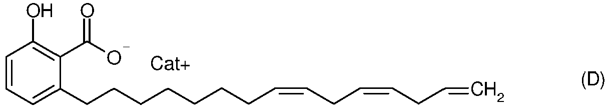 et plus particulièrement les composés **(IIA), (IIB), (IIC)** et **(IID)** sont un mélange dans lequel :
- la proportion du composé (A) varie de 0,001 à 5%,
- la proportion du composé (B) varie de 20 à 45%,
- la proportion du composé (C) varie de 5 à 35%,
- la proportion du composé (D) varie de 25 à 50%, les pourcentages étant en masse par rapport à la masse totale du mélange des composés (A), (B), (C) et (D).

10. Utilisation selon l'une quelconque des revendications 1 à 9, **caractérisée en ce que** les composés **(IIA), (IIB), (IIC), (IID),** sont choisis parmi les composés **1, 2, 3, 4, 5, 6, 7, 8:** 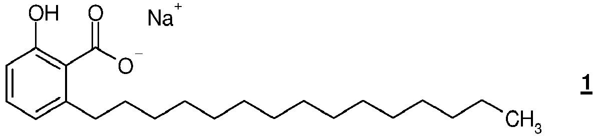 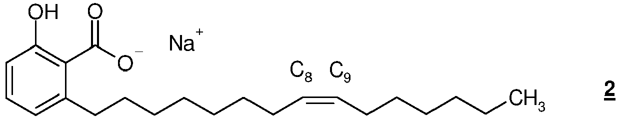 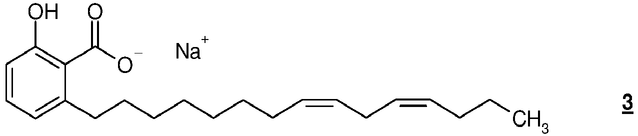 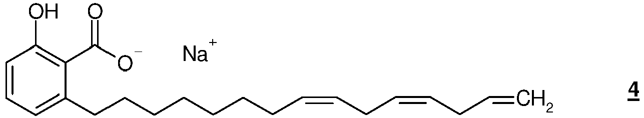 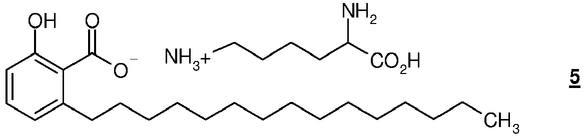 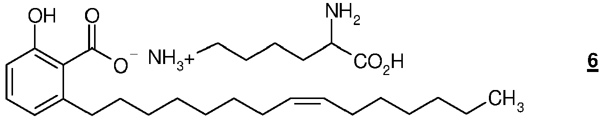 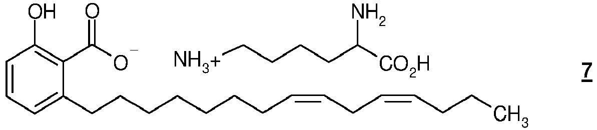 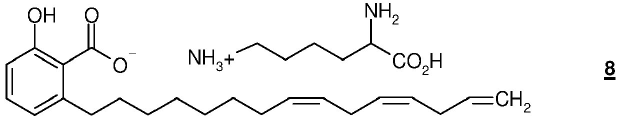 et plus préférentiellement les composés **(IIA), (IIB), (IIC), (IID)** sont un mélange des composés **1, 2, 3** et **4** ou un mélange des composés **5, 6, 7** et **8,** et encore plus préférentiellement un mélange dans lequel les **composés 1, 2, 3, 4** ou les composés **5, 6, 7 et 8** sont dans les mêmes proportions que respectivement les composés (A), (B), (C) et (D) de la revendication 7.

11. Utilisation selon la revendication 10, **caractérisée en ce que** les composés **(IIA), (IIB), (IIC), (IID)** sont choisis parmi les mélanges suivants :
(i) un mélange comprenant 5% en masse de composé **1,** 35% de composé **2,** 23% en masse de composé **3** et 37% de composé **4** ;
(ii) un mélange comprenant 5% en masse de composé **5,** 35% de composé **6,** 23% en masse de composé **7** et 37% de composé **8.**

12. Composition comprenant dans un milieu physiologiquement acceptable :
i) un mélange de composés de formule (II) ou l'une de leurs formes tautomères, leurs isomères optiques, leurs isomères géométriques ou leurs solvates, tel que défini selon l'une quelconque des revendications 1 à 11,
ii) au moins un actif anti-transpirant et/ou au moins un autre actif déodorant.

13. Dispositif aérosol constitué par :
(A) au moins un récipient comprenant une composition telle que définie selon la revendication 12.
(B) au moins un agent propulseur et au moins un moyen de distribution de ladite composition sous forme aérosol.

14. Méthode cosmétique de traitement des odeurs corporelles, en particulier axillaires, consistant à appliquer sur la surface d'une matière kératinique humaine une composition selon la revendication 12.
